# FASCICULE DE BREVET EUROPEEN

(11) **EP 0 727 971 B1**
(45) Date de publication et mention de la délivrance du brevet: **14.01.1998**
(21) Numéro de dépôt: 95901472.1
(22) Date de dépôt: 09.11.1994
(51) Int. Cl.: A61F 9/00

(54) **INSTRUMENT CHIRURGICAL DU TYPE PINCE A EMPORTE-PIECE, DESTINE A LA CHIRURGIE OCULAIRE**
LOCHZANGENARTIGES CHIRURGISCHES INSTRUMENT BESTIMMT FÜR AUGENCHIRURGIE
PUNCH FORCEPS TYPE SURGICAL INSTRUMENT FOR EYE SURGERY

(30) Priorité: 10.11.1993 FR 9313439
(43) Date de publication de la demande: 28.08.1996
(73) Titulaire: MORIA S.A., F-75006 Paris (FR); De Laage de Meux, Patrice, 75116 Paris (FR); Crozafon, Philippe, F-06000 Nice (FR)
(72) Inventeur: DE LAAGE DE MEUX, Patrice, F-75116 Paris (FR); CROZAFON, Philippe, F-06000 Nice (FR)
(74) Mandataire: Jaunez, Xavier
(86) Numéro de dépôt international: FR9401311
(87) Numéro de publication internationale: WO9513036

(56) Documents cités:
- EP-A- 0 244 491
- EP-A- 0 517 252
- WO-A-82/03168
- WO-A-91/08784
- DE-U- 8 518 482
- GB-A- 2 022 421
- US-A- 4 509 516
- DATABASE WPI Section PQ, Week 9319, Derwent Publications Ltd., London, GB; Class P, AN 93-157600 & SU,A,1 734 735 (EYE MICROSURGERY RES.) cité dans la demande
- DATABASE WPI Section PQ, Week 9411, Derwent Publications Ltd., London, GB; Class P, AN 94-089999 & SU,A,2 003 353 (DON MED. INST.)

## Description

L'invention concerne le domaine de l'instrumentation chirurgicale, et plus particulièrement les instruments du type pinces à emporte-pièce destinés à la chirurgie oculaire, en vue d'effectuer une trabéculectomie associée à l'opération de la cataracte.

La trabéculectomie est une intervention chirurgicale qui est fréquemment effectuée pour le traitement de glaucomes.

Le glaucome ou hypertension oculaire est une affection oculaire grave qui se traduit par une élévation de la pression intra-oculaire due à une hypersécrétion d'humeur aqueuse ou à un drainage naturel insuffisant. Cette affection constitue la première cause de cécité, de sorte que son traitement fait l'objet de recherches permanentes.

Le traitement des glaucomes est en général purement médical au début (en utilisant des substances telles que des bêta-bloquants), mais le caractère évolutif de cette pathologie conduit souvent à la solution chirurgicale qui consiste à réaliser une trabéculectomie.

La trabéculectomie est d'ailleurs souvent associée à l'opération de la cataracte, bien que les deux affections aient des causes très différentes. Dans ce cas, la séquence opératoire consiste d'abord à opérer la cataracte, puis à procéder à la trabéculectomie.

D'une façon générale, la trabéculectomie est une opération chirurgicale qui consiste à réaliser une perforation du trabéculum, afin de permettre l'écoulement de l'humeur aqueuse qui est en surpression dans la chambre antérieure de l'oeil, et de réguler ainsi la pression intra-oculaire.

La technique traditionnellement utilisée consiste à ouvrir la conjonctive et à réaliser un volet ou trappe dans la sclérotique, afin de pouvoir perforer le trabéculum (voir par exemple les documents WO-A-82 03168 de SU-A-1 734 735).

On utilise en général pour cette ablation un instrument coupant tel que bistouri, ciseaux, ou pince à emporte-pièce, ce dernier instrument étant souvent dénommé "punch" par les spécialistes.

La pince à emporte-pièce alors utilisée comporte un corps allongé sur lequel est articulée une pédale allongée, ainsi qu'un emporte-pièce constitué par une lame tubulaire rigidement fixée en bout du corps, et par un piston coulissant dans cette lame tubulaire, piston dont l'extrémité distale présente une encoche à bord tranchant et dont l'extrémité proximale s'accroche en bout de la pédale.

La pince est rappelée par une lame élastique dans sa position de repos dans laquelle les branches de manoeuvre (en arrière de l'articulation) sont écartées : dans cette position, l'encoche à bord tranchant dépasse du bord de coupe circulaire de la lame tubulaire. Lorsque le chirurgien serre les branches de manoeuvre, la pédale rétracte le piston de coupe à l'intérieur de la lame tubulaire, et le tissu est pincé entre le bord de coupe circulaire de la lame tubulaire et le bord tranchant de l'encoche du piston de coupe, ce qui réalise à la coupe du tissu.

Il convient de noter que ce type de pince à emporte-pièce est conçu pour effectuer une perforation avec découpe, et diffère de ce fait notablement des pinces à emporte-pièce utilisées dans d'autres domaines de la chirurgie et conçues pour effectuer des prélèvements de tissus ou de fragments osseux (de telles pinces de prélèvement sont par exemple illustrées dans les documents EP-A-0 244 491, GB-A-2 022 421 et DE-U-85 18 482).

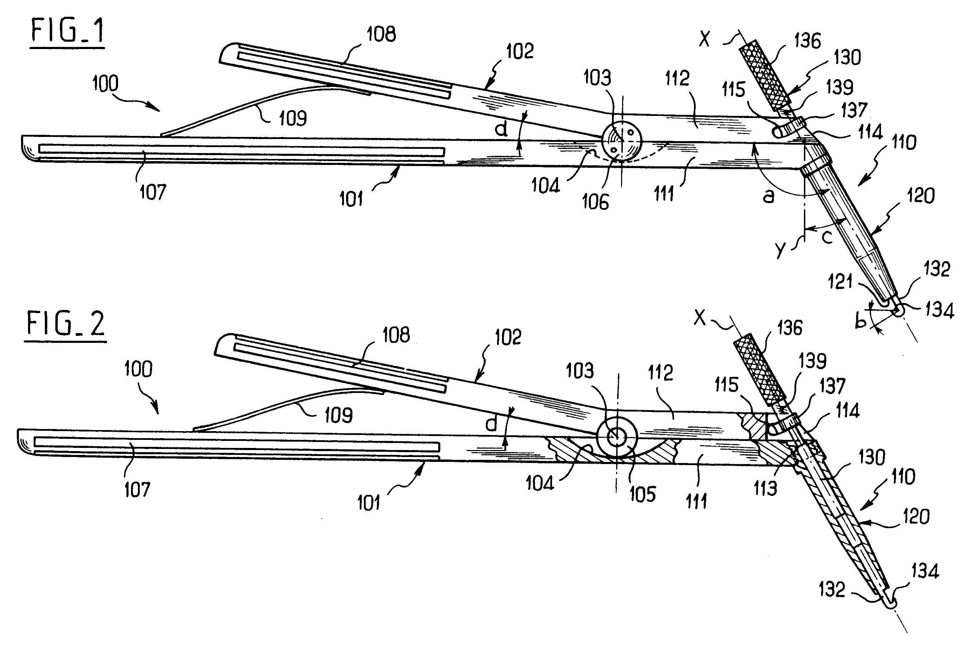

On a illustré schématiquement sur la figure 1O le mode habituel d'utilisation, en vue d'une trabéculectomie, d'une pince à emporte-pièce du type précité.

Sur cette figure, un oeil 1 a été représenté avec sa cornée 2 recouverte de la conjonctive 3, sa sclérotique 4 (ou sclère). On distingue également l'iris 5, la chambre antérieure 6 occupée par l'humeur aqueuse, le cristallin 7 et la zonule 8 qui s'y rattache, et la chambre postérieure 9 occupée par l'humeur vitrée. La zone annulaire 1O comporte les muscles zonulaires et le trabéculum.

Après avoir ouvert la conjonctive, le chirurgien découpe alors un volet 11 dans l'épaisseur externe de la sclère, d'environ 5 à 7 mm de côté, et pose un fil 19 pour maintenir ce volet ouvert. Une fenêtre 12 est ensuite découpée dans l'épaisseur interne de la sclère, de façon à permettre d'accéder aux tissus du trabéculum. Avec une pince (non représentée), une partie du trabéculum est tirée, puis découpée avec des ciseaux ou, comme illustré ici, avec une pince à emporte-pièce 2O.

On a schématisé par 13 l'axe de l'emporte-pièce, qui est constitué d'une lame tubulaire 15 à bord libre de coupe 17, et d'un piston de coupe 14 se terminant par une encoche à bord tranchant 16. La direction du corps de l'instrument 2O est schématisée par la ligne en traits mixtes 18.

Il est à noter que l'axe 13 est perpendiculaire à la direction 18, et que le bord tranchant 16 du piston de coupe est droit, c'est-à-dire perpendiculaire à l'axe du piston.

Le travail du chirurgien doit s'effectuer sous microscope (schématisé en M), de sorte que l'accès à la zone d'intervention est de facto limité. Le chirurgien ne dispose de ce fait que d'une liberté assez restreinte pour la position et le maniement de l'instrument, et la direction d'approche de l'emporte-pièce est alors essentiellement orthogonale, comme illustré sur la figure 1O.

La trabéculectomie est éventuellement suivie d'une iridectomie, après quoi le chirurgien referme le volet scléral et en effectue la suture.

Cette intervention est délicate et reste encore relativement traumatisante pour le patient. Le suturage est en outre important.

Il serait intéressant de pouvoir accéder différemment au trabéculum pour en effectuer la perforation, mais malheureusement la géométrie de l'outil et la présence du microscope empêchent pratiquement d'envisager d'autre voies d'accès.

L'invention vise précisément à résoudre ce problème, en réalisant un instrument chirurgical qui permet de réaliser une trabéculectomie ne nécessitant pas la réalisation d'un volet scléral.

L'invention a ainsi pour objet de réaliser un instrument chirurgical du type pince à emporte-pièce, dont la structure permet de réaliser une trabéculectomie, de façon simple et peu traumatisante, en passant par une incision tunnelisée du type de celle que l'on réalise pour l'opération de la cataracte.

Il s'agit plus particulièrement d'un instrument chirurgical du type pince à emporte-pièce, destiné à la chirurgie oculaire et étant conçu pour effectuer une trabéculectomie, comportant un corps allongé sur lequel est articulée une pédale allongée, ainsi qu'un emporte-pièce constitué par une lame tubulaire rigidement fixée en bout du corps, et par un piston de coupe coulissant dans cette lame tubulaire, piston dont l'extrémité distale présente une encoche à bord tranchant et dont l'extrémité proximale s'accroche en bout de la pédale, caractérisé en ce que :
- la lame tubulaire de l'emporte-pièce est montée inclinée vers l'avant, de façon à définir avec le corps un angle obtus essentiellement compris entre 95° et 160° en vue de réaliser une trabéculectomie en passant par une incision tunnelisée ;
- le piston de coupe présente un bout terminal de forme ogivale arrondie, afin de faciliter l'introduction de l'emporte-pièce par l'incision tunnelisée ;
- l'encoche du piston de coupe de l'emporte-pièce présente un bord tranchant qui est relevé, de façon à pouvoir accrocher le tissu à couper lors du recul dudit piston.

De préférence, l'axe de la lame tubulaire est rectiligne et dans le plan médian de l'instrument qui est orthogonal à l'axe d'articulation de la pédale, et l'axe de la lame tubulaire forme un angle d'environ 12O° avec la direction générale dans laquelle s'étend le corps.

On obtiendra alors un montage simple et précis en prévoyant que le corps présente un taraudage d'extrémité pour une fixation démontable de la lame tubulaire, l'axe dudit taraudage coïncidant avec l'axe de ladite lame.

Avantageusement encore, le bord tranchant de l'encoche du piston de coupe est relevé par rapport à une direction orthogonale à l'axe du piston, d'un angle essentiellement compris entre 15° et 6O°, et de préférence voisin de 3O°.

Il est en outre intéressant que le bord tranchant de l'encoche soit maintenu dans le plan médian de l'instrument par un guidage associé au moyen d'accrochage de l'extrémité proximale du piston de coupe sur la pédale.

Selon un mode d'exécution avantageux, la pédale se termine par deux ailettes disposées de part et d'autre du plan médian de l'instrument, ces deux ailettes présentant chacune une encoche débouchante incurvée formant le moyen d'accrochage de l'extrémité proximale du piston de coupe par coopération entre ces deux encoches et une saillie cylindrique du piston passant entre lesdites ailettes.

Avantageusement alors, le piston de coupe présente, de part et d'autre de son axe, deux plats formant facettes de guidage par coopération avec les deux ailettes de la pédale, le bord tranchant de l'encoche du piston pouvant alors être disposé dans l'une ou l'autre de deux positions symétriques par rapport à l'axe dudit piston.

Selon une autre caractéristique intéressante, le piston de coupe est au moins en partie interchangeable et à usage unique. Par exemple, l'extrémité distale du piston de coupe est un embout rapporté qui constitue une pièce interchangeable et à usage unique.

D'autres caractéristiques et avantages de l'invention apparaîtront plus clairement à la lumière de la description qui va suivre et des dessins annexés, concernant un mode de réalisation particulier, en référence aux figures où :
- la figure 1 est une vue en élévation d'un instrument chirurgical conforme à l'invention, dans sa position de repos (bord tranchant dépassant du bord de coupe de la lame tubulaire) ;
- les figures 2 et 3 sont deux vues analogues, avec des coupes partielles dans la zone de l'articulation et de l'emporte-pièce, montrant deux orientations possibles du bord tranchant par rapport au corps de l'instrument ;
- la figure 4 illustre une position active de l'instrument précédent ;
- la figure 5 est une coupe axiale, à plus grande échelle, de la lame tubulaire de l'instrument précité ;
- les figures 6 et 7 sont deux vues en élévation (à 90° l'une de l'autre) du piston de coupe du même instrument, permettant notamment de mieux distinguer les deux plats de guidage dans la zone d'accrochage dudit piston ;
- la figure 8 illustre une variante du piston de coupe, à embout rapporté ;
- la figure 9, qui est à rapprocher de la figure 1O déjà décrite relative à la technique antérieure, illustre la technique opératoire de trabéculectomie mise en oeuvre avec un instrument chirurgical conforme à l'invention.

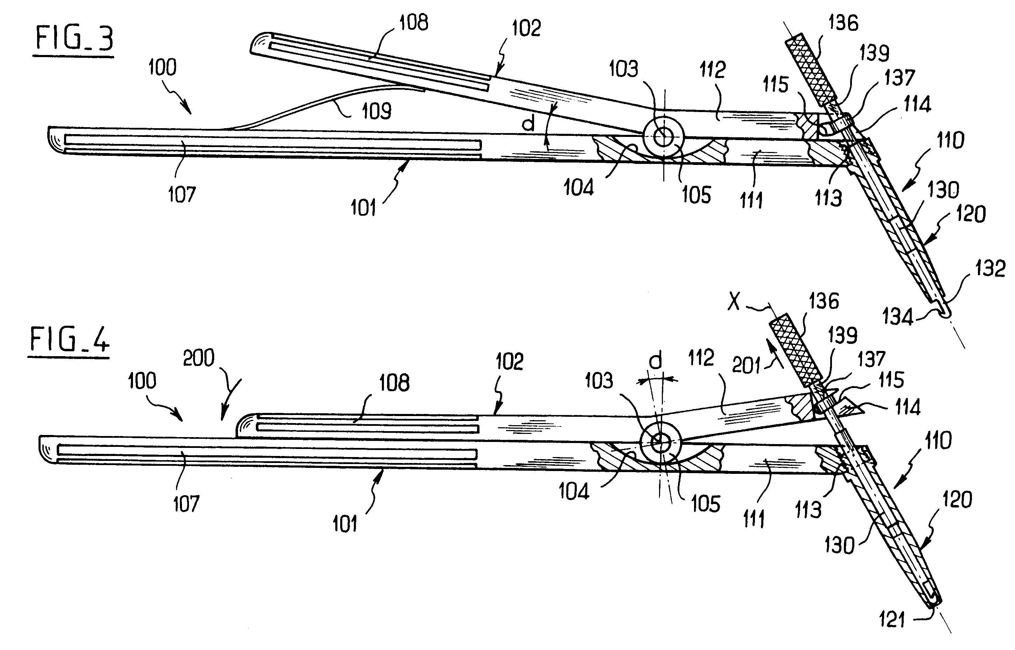

Sur la figure 1, on distingue un instrument du type pince à emporte-pièce 100 conforme à l'invention, comportant un corps allongé 101 sur lequel est articulée une pédale allongée 102, l'axe d'articulation étant noté 103. Les parties arrière du corps et de la pédale respectivement 107 et 108, forment des branches de manoeuvre, tandis que les parties avant 111 et 112 servent au montage de l'emporte-pièce associé 110. Ainsi que cela est mieux visible sur les figures 2 et 3, la pédale 102 présente un disque central 105 qui est reçu dans une fente associée 104 du corps 101, entre deux oreilles latérales (non visibles ici) dudit corps, l'ensemble étant fixé par un moyeu vissé 106. Ce montage garantit le maintien des deux éléments mobiles dans un même plan médian qui est orthogonal à l'axe d'articulation de la pédale. L'instrument chirurgical 100 est rappelé dans sa position de repos par une lame ressort 109 ou tout autre moyen équivalent. Dans cette position, les branches arrières 107 et 108 forment un angle d, dont la valeur est naturellement fonction de la longueur du bras de levier et de la course du piston de coupe de l'emporte pièce, et une valeur moyenne se situera en général dans une plage allant de 15 à 45°. Les parties avant 111 et 112 sont ici jointives dans cette position de repos.

L'instrument chirurgical 100 comporte un emporte-pièce 110 constitué par une lame tubulaire 120 rigidement fixée en bout du corps 101, et par un piston de coupe 130 coulissant dans cette lame tubulaire, piston dont l'extrémité distale présente une encoche à bord tranchant et dont l'extrémité proximale s'accroche au bout de la pédale 102.

Conformément à une première caractéristique de l'invention, la lame tubulaire 120 de l'emporte-pièce 110 est montée inclinée vers l'avant, de façon à définir avec le corps 101 un angle a obtus, essentiellement compris entre 95° et 160°. En l'espèce, l'axe X de la lame tubulaire 120 est rectiligne, et dans le plan médian de l'instrument qui est orthogonal à l'axe d'articulation de la pédale 102, et cet axe X forme un angle a d'environ 120° avec la direction générale dans laquelle s'étend le corps 101.

Ainsi que cela est mieux visible sur les figures 2 à 4, on constate que le corps 1O1 présente un taraudage d'extrémité 113 pour une fixation démontable de la lame tubulaire 120, l'axe dudit taraudage coïncidant naturellement avec l'axe X de cette lame tubulaire. Il va de soi que ce type de montage pourra être remplacé par tout autre moyen garantissant l'orientation de l'emporte-pièce, et permettant l'interchangeabilité de celui-ci.

Ainsi, l'emporte-pièce de l'instrument chirurgical conforme à l'invention est incliné vers l'avant d'un angle c au-delà d'une direction perpendiculaire Y qui était celle des instruments chirurgicaux de l'art antérieur, l'angle c allant de 5 à 70°, et étant en l'espèce voisin de 30°.

Cette première angulation entre l'axe d'emporte-pièce et la direction générale du corps d'instrument, permet d'envisager de réaliser une trabéculectomie en passant par une incision tunnelisée du type de celle que l'on réalise pour l'opération de la cataracte.

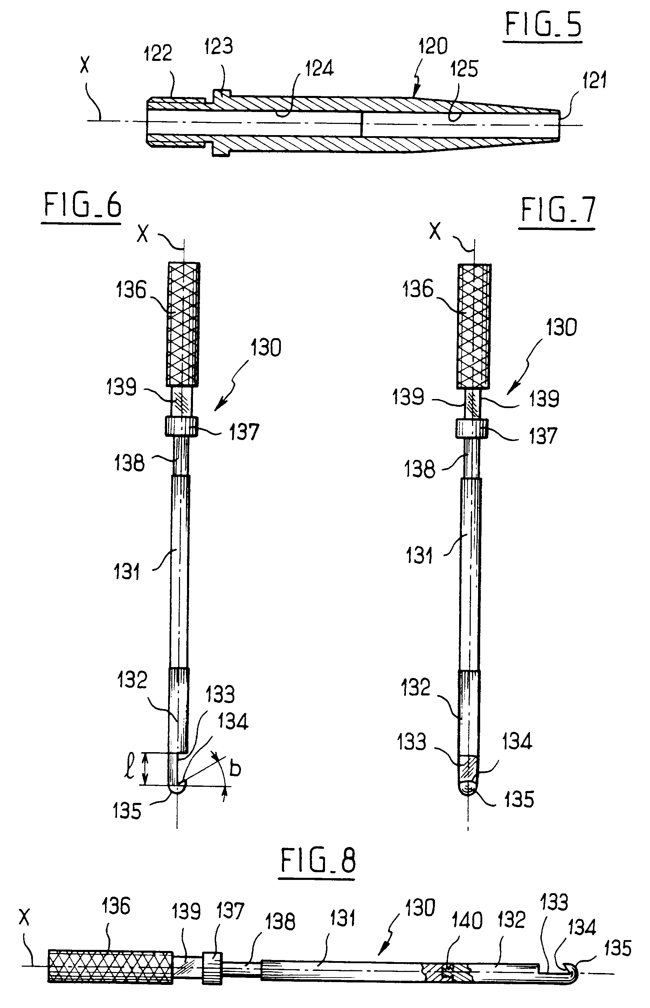

En outre, et selon une deuxième caractéristique de l'invention, le piston de coupe 130 présente un bout terminal 135 (mieux visible sur les figures 6 à 8) de forme ogivale arrondie, afin de faciliter l'introduction douce de l'emporte-pièce 110 par l'incision tunnelisée, sans risque de coupure de tissus.

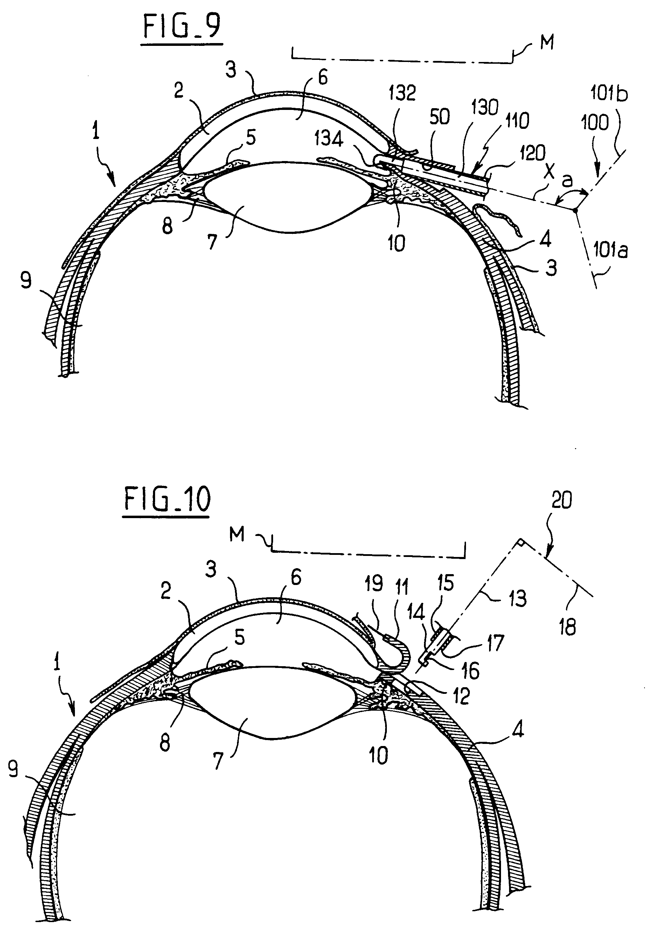

Sur la figure 9, les différentes parties de l'oeil référencées de 1 à 1O correspondent à celles déjà mentionnées précédemment en référence à la figure 1O. On constate cependant que le chirurgien, après avoir ouvert la conjonctive 3, a effectué une incision tunnellisée 5O du type de celle habituellement réalisée pour l'opération de la cataracte, en utilisant un couteau-diamant (non représenté ici) dont la largeur (en général située entre 2,8 mm et 3,2 mm) donne le calibrage de l'incision. L'incision tunnellisée 50 est effectuée jusqu'à pouvoir déboucher dans la chambre antérieure 6 de l'oeil. Le chirurgien peut alors mettre en place l'instrument chirurgical 100 conforme à l'invention, dans l'une ou l'autre des deux positions notées 101a et 101b du corps de cet instrument, de façon à être à sa main. Sur cette figure 9, on a seulement schématisé par des traits mixtes la présence du corps de l'instrument, et l'on reconnaît la partie terminale de l'emporte-pièce 110 précédemment décrite, qui est insérée dans l'incision tunnellisée 50. L'emporte-pièce est ainsi avancé dans l'incision tunnellisée jusqu'à ce que l'extrémité distale 132 du piston de coupe de l'instrument pénètre dans la chambre antérieure 6. Il est important de noter que dans le cadre d'une telle mise en place, le chirurgien fonctionne en aveugle, dans la mesure où la sclère 4 est opaque, et où il peut tout juste apercevoir, à travers la cornée, l'extrémité terminale du piston de coupe. Ceci complique naturellement le travail de la coupe, car le chirurgien doit s'arranger pour saisir le tissu à couper sans exercer de tension sur l'incision de la cataracte.

Ce problème est résolu conformément à l'invention, grâce à une troisième caractéristique essentielle de l'invention, selon laquelle l'encoche du piston de coupe 130 de l'emporte-pièce présente un bord tranchant 134 qui est relevé, de façon à pouvoir accrocher le tissu à couper lors du recul dudit piston. Ainsi, le bord tranchant n'est plus un bord droit comme c'était le cas pour les techniques antérieures, mais un bord relevé formant une sorte d'hameçon qui peut accrocher le tissu à couper lors du mouvement de recul du piston de coupe. Grâce à cet accrochage, le chirurgien peut effectuer une découpe précise de la paroi interne de la sclère et du trabéculum, malgré l'absence du contrôle de cette découpe par le microscope M. Une fois la trabéculectomie terminée, il suffit alors au chirurgien de retirer l'emporte-pièce, et de refermer l'incision tunnellisée de la sclère, incision dont la largeur permet même d'envisager une absence de suturage pour sa refermeture. L'intervention est donc considérablement simplifiée, et elle est beaucoup moins traumatisante pour le patient ; le suturage est en outre minime, voire inutile, dans la mesure où l'on utilise une incision tunnellisée d'environ 2,5 mm de largeur.

L'instrument chirurgical selon l'invention peut donc être utilisé pour une intervention anti-glaucomateuse isolée de trabéculectomie, ou encore postérieurement à une opération de la cataracte, par exemple par phacoémulsification, en passant alors par l'incision tunnellisée déjà pratiquée pour cette opération préliminaire.

Le chirurgien peut ainsi effectuer une trabéculectomie isolée ou subséquente à une opération de la cataracte en effectuant une incision tunnellisée en partie haute de l'oeil, de sorte qu'il est parfaitement à sa main pour la mise en place de l'instrument chirurgical dans la mesure où il se trouve derrière la tête du patient couché.

Dans le cadre des techniques antérieures, on procédait en général à une incision tunnellisée dans la partie haute de l'oeil pour la cataracte, mais, pour la trabéculectomie, on procédait à la réalisation d'un volet scléral en-dessous de la cornée : l'invention apporte à ce niveau un avantage très important. Naturellement, si cela s'avère nécessaire, une iridectomie peut être pratiquée avant la refermeture de l'incision sclérale tunnellisée linéaire. L'instrument selon l'invention permet ainsi de réaliser instantanément une opération filtrante protégée analogue à une trabéculectomie, en introduisant l'emporte-pièce dans la chambre antérieure de l'oeil, et en revenant très légèrement en arrière afin d'accrocher le plan plafond de la cornée périphérique, puis en réalisant une découpe précalibrée demi-circulaire d'environ 1,7 mm de diamètre, sans avoir à modifier la position de l'instrument.

L'angulation de l'emporte-pièce confère au chirurgien une aisance importante et une grande précision du geste (le corps de l'instrument sera en général orienté vers le haut, comme schématisé par la position 101b sur la figure 9). La géométrie spécifique de l'extrémité distale du piston de coupe aide ainsi à une introduction atraumatique et directe dans la chambre antérieure, sans avoir ainsi à soulever le trapon scléral. La forme particulière de la partie tranchante du piston de coupe assure immanquablement d'être au contact de l'angle irido-cornéen, et d'exciser une quantité toujours égale de tissu scléro-cornéen.

Les figures 5 à 7 permettent de mieux distinguer la structure de la lame tubulaire 120 et du piston de coupe 130 de l'instrument chirurgical précité.

La lame tubulaire 120 comporte un alésage central, ici en deux parties coaxiales 124, 125, se terminant par un bord libre de coupe 121 du côté distal. Pour l'extrémité proximale, on trouve un embout fileté 122 permettant le montage de la lame en extrémité du corps de l'instrument, avec une collerette de butée 123.

Le piston de coupe 130 comporte quant à lui un corps central cylindrique 131, auquel se raccorde l'extrémité distale 132 présentant une encoche 133 dont le bord tranchant 134 est relevé d'un angle b, dont on a vu qu'il était essentiellement compris entre 15° et 60°, et de préférence voisin de 30°. L'angle b doit en effet être suffisant pour accrocher le tissus à couper, mais il ne doit pas être trop grand pour éviter d'accrocher d'autres tissus lors de la coupe. La hauteur l de l'encoche 133 sera dans la pratique de l'ordre de 1 à 3 mm : on évitera une encoche plus haute, afin de ne pas avoir une course trop longue qui présenterait les risques de toucher l'iris ou d'endommager les tissus à l'arrière.

Il est par ailleurs important que le bord tranchant 134 de l'encoche puisse être maintenu dans le plan médian de l'instrument, afin d'éviter toute imprécision lors de la coupe. Ceci est obtenu conformément à l'invention, grâce à un guidage associé au moyen d'accrochage de l'extrémité proximale du piston de coupe sur la pédale. En effet, si l'on se reporte aux figures 1 à 4, on constate que la pédale 102 se termine par deux ailettes 114 disposées de part et d'autre du plan médian de l'instrument, ces deux ailettes présentant chacune une encoche débouchante incurvée 115 formant le moyen d'accrochage de l'extrémité proximale du piston de coupe par coopération entre ces deux encoches 115 et une saillie cylindrique 137 du piston passant entre lesdites ailettes. L'accrochage de la saillie cylindrique 137 garantit la précision du mouvement axial du piston de coupe lorsque le chirurgien serre les deux branches de manoeuvre de l'instrument. Pour le maintien d'une position angulaire précise et stable, on a prévu, ainsi que cela est mieux visible sur les figures 6 et 7, que le piston de coupe 130 présente, de part et d'autre de son axe X, deux plats 139 formant facettes de guidage par coopération avec les faces internes des deux ailettes 114 de la pédale 102. Comme illustré aux figures 2 et 3, le bord tranchant 134 de l'encoche du piston peut être alors disposé dans l'une ou l'autre de deux positions symétriques par rapport à l'axe dudit piston. Pour changer de position, il suffit de démonter, en la dévissant, la lame tubulaire 120, pour dégager le piston de coupe 130, de préférence en le saisissant par sa partie terminale moletée 136, puis d'effectuer une rotation de 180° du piston autour de son axe X, et de remonter la lame tubulaire sur le corps de l'instrument. Ainsi, la saillie cylindrique 137 du piston de coupe est bordée supérieurement par les deux plats de guidage 139, et inférieurement par une portion cylindrique 138 dont le diamètre correspond à la distance entre les deux facettes de guidage précitées. Ceci permet d'avoir un écartement constant entre les deux faces internes des ailettes 114.

Comme représenté sur la figure 4, lorsque le chirurgien serre l'instrument 100, selon la flèche 200, il réalise un recul du piston de coupe 130 conformément à la flèche 201, pour la coupe désirée du tissu par l'emporte-pièce dudit instrument.

Il est par ailleurs intéressant de prévoir que le piston de coupe 130 est au moins en partie interchangeable et à usage unique, c'est-à-dire constitue un composant jetable. En effet, grâce à cette mesure, on est certain de toujours disposer d'un tranchant parfaitement affûté, et d'éviter une déchirure des tissus plutôt qu'une coupe franche de ceux-ci. On pourra alors réaliser le piston de coupe en matière plastique ou en métal tendre. On pourra également en variante, comme cela est illustré sur la figure 8, prévoir que l'extrémité distale 132 du piston de coupe est un embout rapporté qui constitue une pièce interchangeable et à usage unique, cet embout étant par exemple mis en place par vissage d'un pion fileté 140 dans un taraudage associé du corps de piston.

On est ainsi parvenu à réaliser un instrument chirurgical permettant d'effectuer une trabéculectomie ne nécessitant pas la réalisation d'un volet scléral, en passant par une incision tunnellisée du type de celle que l'on réalise pour l'opération de la cataracte. L'instrument chirurgical ainsi conçu présente une structure simple et légère, et parfaitement maniable, et la précision de son maniement permet au chirurgien d'opérer en toute tranquillité malgré un travail en aveugle pour la perforation du trabéculum.

L'invention n'est pas limité au mode de réalisation qui vient d'être décrit, mais englobe au contraire toute variante reprenant, avec des moyens équivalents, les caractéristiques essentielles énoncées plus haut, l'invention étant limitée seulement par les revendications.

## Revendications

1. Instrument chirurgical du type pince à emporte-pièce, destiné à la chirurgie oculaire et étant conçu pour effectuer une trabéculectomie, comportant un corps allongé (101) sur lequel est articulée une pédale allongée (102), ainsi qu'un emporte-pièce (110) constitué par une lame tubulaire (120) rigidement fixée en bout du corps (101), et par un piston de coupe (130) coulissant dans cette lame tubulaire, piston dont l'extrémité distale présente une encoche (133) à bord tranchant et dont l'extrémité proximale s'accroche en bout de la pédale (102), caractérisé en ce que :
- la lame tubulaire (12O) de l'emporte-pièce (110) est montée inclinée vers l'avant, de façon à définir avec le corps (101) un angle (a) obtus essentiellement compris entre 95° et 160°, en vue de réaliser une trabéculectomie en passant par une incision tunnelisée (50) ;
- le piston de coupe (130) présente un bout terminal (135) de forme ogivale arrondie, afin de faciliter l'introduction de l'emporte-pièce (110) par l'incision tunnelisée (50) ;
- l'encoche (133) du piston de coupe (13O) de l'emporte-pièce (11O) présente un bord tranchant (134) qui est relevé, de façon à pouvoir accrocher le tissu à couper lors du recul dudit piston.

2. Instrument chirurgical selon la revendication 1, caractérisé en ce que l'axe (X) de la lame tubulaire (12O) est rectiligne et dans le plan médian de l'instrument qui est orthogonal à l'axe d'articulation de la pédale (1O2).

3. Instrument chirurgical selon les revendications 1 et 2, caractérisé en ce que l'axe (X) de la lame tubulaire (12O) forme un angle (a) d'environ 12O° avec la direction générale dans laquelle s'étend le corps (1O1).

4. Instrument chirurgical selon la revendication 2 ou la revendication 3, caractérisé en ce que le corps (1O1) présente un taraudage d'extrémité (113) pour une fixation démontable de la lame tubulaire (12O), l'axe dudit taraudage coïncidant avec l'axe (X) de ladite lame.

5. Instrument chirurgical selon l'une des revendications 1 à 4, caractérisé en ce que le bord tranchant (134) de l'encoche du piston de coupe (13O) est relevé par rapport à une direction orthogonale à l'axe (X) du piston, d'un angle (b) essentiellement compris entre 15° et 6O°, et de préférence voisin de 3O°.

6. Instrument chirurgical selon l'une des revendications 2 à 5, caractérisé en ce que le bord tranchant (134) de l'encoche est maintenu dans le plan médian de l'instrument par un guidage associé au moyen d'accrochage (114, 115) de l'extrémité proximale (137) du piston de coupe (13O) sur la pédale (1O2).

7. Instrument chirurgical selon l'une des revendications 2 à 6, caractérisé en ce que la pédale (1O2) se termine par deux ailettes (114) disposées de part et d'autre du plan médian de l'instrument, ces deux ailettes présentant chacune une encoche débouchante incurvée (115) formant le moyen d'accrochage de l'extrémité proximale du piston de coupe par coopération entre ces deux encoches (115) et une saillie cylindrique (137) du piston passant entre lesdites ailettes.

8. Instrument chirurgical selon les revendications 6 et 7, caractérisé en ce que le piston de coupe (13O) présente, de part et d'autre de son axe, deux plats (139) formant facettes de guidage par coopération avec les deux ailettes (114) de la pédale (1O2), le bord tranchant (134) de l'encoche du piston pouvant alors être disposé dans l'une ou l'autre de deux positions symétriques par rapport à l'axe dudit piston.

9. Instrument chirurgical selon l'une des revendications 1 à 8, caractérisé en ce que le piston de coupe (13O) est au moins en partie interchangeable et à usage unique.

10. Instrument chirurgical selon la revendication 9, caractérisé en ce que l'extrémité distale (132) du piston de coupe (13O) est un embout rapporté qui constitue une pièce interchangeable et à usage unique.

## Claims

1. A surgical instrument of the forceps punch type for eye surgery and being designed to perform trabeculectomy, the instrument having an elongate body (101) on which an elongate lever (102) is hinged, together with a punch (110) constituted by a tubular blade (120) rigidly fixed to the end of the body (101) and by a cutting plunger (130) slidably mounted in said tubular blade, the distal end of the plunger having a notch (133) with a cutting edge and the proximal end of the plunger being connected to the end of the lever (102), the instrument being characterized in that:
• the tubular blade (120) of the punch (110) is mounted so as to be forwardly inclined, thereby defining an obtuse angle (a) relative to the body (101) lying essentially in the range 95° to 160°, in order to perform trabeculectomy by passing through a tunnel incision (50) ;
• the cutting plunger (130) has a terminal end (135) that has a rounded shape in order to facilitate insertion of the punch through the tunnel incision (50) ; and
• the notch (133) of the cutting plunger (130) of the punch (110) has a cutting edge (134) which is raised, so as to enable it to hook onto the tissue to be cut when said plunger is withdrawn.

2. A surgical instrument according to claim 1, characterized in that the axis X of the tubular body (120) is rectilinear and lies in the midplane of the instrument which is orthogonal to the hinge axis of the lever (102).

3. A surgical instrument according to claim 1 or 2, characterized in that the axis (X) of the tubular blade (120) forms an angle (a) of about 120° with the general direction in which the body (101) extends.

4. A surgical instrument according to claim 2 or claim 3, characterized in that the body (101) has an end tapping (113) for dismountably securing the tubular blade (120), the axis of said tapping coinciding with the axis (X) of said blade.

5. A surgical instrument according to any one of claims 1 to 4, characterized in that the cutting edge (134) of the notch of the cutting plunger (130) is raised relative to the orthogonal to the axis (X) of the plunger through an angle (b) lying essentially between 15° and 60°, and preferably about 30°.

6. A surgical instrument according to any one of claims 2 to 5, characterized in that the cutting edge (134) of the notch is held in the midplane of the instrument by guidance associated with the means (114, 115) for fastening the proximal end (137) of the cutting plunger (130) to the lever (102).

7. A surgical instrument according to any one of claims 2 to 6, characterized in that the lever (102) is terminated by two fins (114) disposed on either side of the midplane of the instrument, each of these two fins having a respective curved open-ended notch (115) forming the means for fastening the proximal end of the cutting plunger by co-operation between these two notches (105) and a cylindrical projection (137) on the plunger which passes between said fins.

8. A surgical instrument according to claims 6 and 7, characterized in that the cutting plunger (130) presents two guidance facet-forming flats (139) on either side of its axis that co-operate with the two fins (114) of the lever (102), the cutting edge (134) of the notch of the plunger then being capable of being disposed in one or other of two symmetrical positions relative to the axis of said plunger.

9. A surgical instrument according to any one of claims 1 to 8, characterized in that the cutting plunger (130) is interchangeable, at least in part, and suitable for single use.

10. A surgical instrument according to claim 9, characterized in that the distal end (132) of the cutting plunger (130) is an add-on endpiece which constitutes an interchangeable part for single use.

## Patentansprüche

1. Lochzangenartiges chirurgisches Instrument für die Augenchirurgie, das zum Durchführen einer Trabekulotomie bestimmt ist, umfassend einen länglichen Körper (101), an dem ein länglicher Hebel (102) angelenkt ist, sowie eine Lochvorrichtung (110), die von einer am Ende des Körpers (101) starr befestigten rohrförmigen Klinge (120) und einem in dieser rohrförmigen Klinge gleitenden Schneidekolben (130) gebildet ist, dessen distales Ende eine mit einem Schneiderand versehene Kerbe (133) hat und dessen proximales Ende an dem Ende des Hebels (102) eingehängt ist, dadurch **gekennzeichnet,**
daß die rohrförmige Klinge (120) der Lochvorrichtung (110) nach vorne geneigt angeordnet ist derart, daß sie mit dem Körper (101) einen stumpfen Winkel (a) von im wesentlichen 95° bis 160° bildet, um eine Trabekulotomie auszuführen, indem man durch einen Tunnelschnitt (50) eindringt,
daß der Schneidekolben (130) ein Ende mit einer Form eines abgerundeten Spitzbogens hat, um das Einführen der Lochvorrichtung (110) in den Tunnelschnitt (50) zu erleichtern,
daß die Kerbe (133) in dem Schneidekolben (130) der Lochvorrichtung (110) einen Schneiderand (134) hat, der ansteigt, um das zu schneidende Gewebe beim zurückziehen des Kolbens hakenförmig erfassen zu können.

2. Chirurgisches Instrument nach Anspruch 1, dadurch **gekennzeichnet**, daß die Achse (X) der rohrförmigen Klinge (120) geradlinig ist und in einer Mittelebene des Instrumentes liegt, die senkrecht zur Gelenkachse des Hebels (102) ist.

3. Chirurgisches Instrument nach den Ansprüchen 1 und 2, dadurch **gekennzeichnet**, daß die Achse (X) der rohrförmigen Klinge (120) einen Winkel (a) von ungefähr 120° mit der allgemeinen Richtung bildet, in der sich der Körper (101) erstreckt.

4. Chirurgisches Instrument nach Anspruch 2 oder 3, dadurch **gekennzeichnet**, daß der Körper (101) an seinem Ende ein Gewinde (113) zur lösbaren Befestigung der rohrförmigen Klinge (120) hat, wobei die Achse dieses Gewindes mit der Achse (X) der Klinge zusammenfällt.

5. Chirurgisches Instrument nach einem der Ansprüche 1 bis 4, dadurch **gekennzeichnet**, daß der Schneiderand (134) der Kerbe des Schneidekolbens (130) gegenüber einer zur Kolbenachse (X) senkrechten Richtung um einen Winkel (b) angehoben ist, der im wesentlichen zwischen 15° und 60°, vorzugsweise annähernd 30° beträgt.

6. Chirurgisches Instrument nach einem der Ansprüche 2 bis 5, dadurch **gekennzeichnet**, daß der Schneiderand (134) der Kerbe durch eine Führung, welche den Verbindungsmitteln (114, 115) zur Verbindung des proximalen Endes (137) des Schneidekolbens (130) mit dem Hebel (102) zugeordnet ist, in der Mittelebene des Instrumentes gehalten wird.

7. Chirurgisches Instrument nach einem der Ansprüche 2 bis 6, dadurch **gekennzeichnet**, daß der Hebel (102) in zwei Schenkel (114) ausläuft, die beiderseits der Mittelebene des Instrumentes angeordnet sind und jeweils eine offene gekrümmte Kerbe (115) haben, welche die Mittel zum Einhängen des proximalen Endes des Schneidekolbens bilden, indem die beiden Kerben (115) mit einem vorspringenden zylindrischen Abschnitt (137) des zwischen den Schenkeln hindurchtretenden Kolbens zusammenwirken.

8. Chirurgisches Instrument nach den Ansprüchen 6 und 7, dadurch **gekennzeichnet**, daß der Schneidekolben (130) beiderseits seiner Achse zwei Abflachungen (139) hat, welche mit den beiden Schenkeln (114) des Hebels (102) zusammenwirkende Führungsflächen bilden, wobei der Schneiderand (134) der an dem Kolben ausgebildeten Kerbe auf diese Weise in der einen oder der anderen von zwei bezüglich der Achse des Kolbens symmetrischen Positionen angeordnet werden kann.

9. Chirurgisches Instrument nach einem der Ansprüche 1 bis 8, dadurch **gekennzeichnet**, daß der Schneidekolben (130) zumindest zum Teil austauschbar und zum einmaligen Gebrauch bestimmt ist.

10. Chirurgisches Instrument nach Anspruch 9, dadurch **gekennzeichnet**, daß das distale Ende (132) des Schneidekolbens (130) ein Ansatzstück ist, welches ein zum einmaligen Gebrauch bestimmtes austauschbares Teil bildet.
